# EUROPEAN PATENT SPECIFICATION

(11) **EP 2 612 659 B1**
(45) Date of publication and mention of the grant of the patent: **06.04.2016**
(21) Application number: 11820998.0
(22) Date of filing: 01.09.2011
(51) Int. Cl.: A61K 9/30, A61K 9/48, A61K 31/704, A61P 9/00, A61K 36/258

(54) **PANAXATRIOL SAPONINS ENTERIC PELLET, CAPSULE COMPRISING SAME AND METHOD FOR PREPARING SAME**
ENTERISCHE PANAXATRIOL-SAPONIN-PILLE, KAPSEL DAMIT UND VERFAHREN ZU IHRER HERSTELLUNG
GRANULE ENTÉRIQUE DE SAPONINES DE PANAXATRIOL, CAPSULE COMPRENANT CELUI-CI ET PROCÉDÉ POUR PRÉPARER CELUI-CI

(30) Priority: 02.09.2010 CN 201010273504
(43) Date of publication of application: 10.07.2013
(73) Proprietor: Pharmaceutical Factory of Chengdu Huasun Group Inc., Ltd., Sichuan 610225 (CN); Chengdu University of TCM Huasun Pharmaceutical Co., Ltd., Sichuan 610072 (CN)
(72) Inventor: WAN, Fang, Chengdu Sichuan 610225 (CN); YANG, Huarong, Chengdu Sichuan 610225 (CN); LIN, Dasheng, Chengdu Sichuan 610225 (CN); CAO, Ke, Chengdu Sichuan 610225 (CN); ZHANG, Wenwu, Chengdu Sichuan 610225 (CN); TANG, Zeng, Chengdu Sichuan 610225 (CN)
(74) Representative: Eisenführ Speiser
(86) International application number: PCT/CN2011/001484
(87) International publication number: WO 2012/027968

(56) References cited:
- CN-A- 1 353 991
- CN-A- 1 698 632
- CN-A- 1 771 978
- CN-A- 101 439 076
- CN-A- 101 612 195
- CN-A- 101 703 546
- DATABASE MEDLINE [Online] US NATIONAL LIBRARY OF MEDICINE (NLM), BETHESDA, MD, US; June 2009 (2009-06), LAI CHUNHUA ET AL: "[Preparation and prescription of enteric coated pellets of Panax notoginseng saponins pellets].", XP002723844, Database accession no. NLM19771862
- LENG JING ET AL.: 'Study on Fingerprints of PTS and its Preparation by LC-MS' WEST CHINA JOURNAL OF PHARMACEUTICAL SCIENCES vol. 24, no. 5, 2009, pages 503 - 505, XP008169427

## Description

### FIELD OF THE INVENTION

The present invention relates to the field of medicine, specifically to a panaxatriol saponins enteric pellet primarily for use in the treatment of cardiovascular and cerebrovascular embolic diseases, a capsule comprising the enteric pellet, and a method for preparing the enteric pellet.

### BACKGROUND OF THE INVENTION

The Sanchi Tongshu Capsule is a marketed former national class II new drug of Chinese materia medica, and is an enteric preparation of effective parts of Panax notoginseng, primarily for the treatment of vascular embolic diseases (such as stroke). The active pharmaceutical ingredients (APIs) of the Sanchi Tongshu Capsule (panaxatriol saponins) are isolated and purified by employing macroporous adsorption resin technology and are controlled for substance group by employing fingerprint technology; the process technology and quality control technology thereof are advanced; the material basis is clear; the pharmaceutically effective ingredients are accurate; the total content of the three main effective ingredients (ginsenoside Rg1, ginsenoside Re, and notoginsenoside R1) achieve more than 70%; the mechanism of action is definite; so that it is a modern Chinese materia medica for the treatment of cardiovascular and cerebrovascular diseases. Panaxatriol saponins are controlled for substance group by employing fingerprint technology, the increased quality standards thereof have been recorded in Part I of "Chinese Pharmacopoeia", 2010 Edition. Since the marketing of the Sanchi Tongshu Capsule, it has been widely recognized by clinical experts and patients, due to its significant curative effects.

The preclinical studies of the Sanchi Tongshu Capsule show that the active ingredient Rg1 of panaxatriol saponins which are the APIs of the Sanchi Tongshu Capsule is easily destroyed under acidic environment of the gastric fluid, and is basically stable under near-neutral environment, and Rg1 is relatively stable in the small intestine. Therefore, in order to ensure the bioavailability of the pharmaceutical active ingredients of the Sanchi Tongshu Capsule, the APIs panaxatriol saponins need to be prepared into an enteric preparation. The current preparation technology of the Sanchi Tongshu Capsule is: mixing panaxatriol saponins powders and carriers, and filling them into empty enteric capsules, to avoid the degradation of the pharmaceutical effective ingredients.

However, during the production and use of the Sanchi Tongshu Capsule, it is found that some technical defects exist:
1) the capsule shell is fragile: the enteric capsule shell appears to be fragile during use, which affects its being taken by a patient, and also affects the effects of positioning release of the enteric preparation, and thus reduces the curative effects of the drug;
2) the empty enteric capsule is unstable in quality: currently, there are very few domestic manufacturers for the empty enteric capsule, only 3 small factories are manufacturing domestically, and foreign manufacturers for empty enteric capsule are hardly found; on the whole, the quality of the empty enteric capsule is inferior to that of a common gastric capsule, and capsule perforation and other phenomena often occur, which causes adverse effects on the production and quality assurance of this product;
3) There are inherent technical defects in the current production technology of the empty enteric capsule: the current production technology of the empty enteric capsule is to coat a enteric coating outside a common gastric capsule shell; due to the special nature of gelatin which is the main ingredient for the capsule shell, this traditional coating manner is hard to achieve desired effects, either affecting the nature of the drug content, or affecting the stability of the entire capsule.

Therefore, the problems of fragile capsule shell, instable quality the empty enteric capsule, etc. have become important issues need to be resolved urgently, which affect the marketing and clinical application of the Sanchi Tongshu Capsule.

CN198632A discloses enteric coated preparations, such as enteric capsules, enteric tablets and enteric pellets, comprising panaxatriol saponins.

### SUMMARY OF THE INVENTION

The purpose of the present invention is to solve the technical defects of the current commercially available Sanchi Tongshu Capsule, to provide an enteric pellet of panaxatriol saponins, i.e. to prepare the panaxatriol saponins into pellets first, then to coat the enteric coating. The enteric pellets can be filled into common gastric capsules, to prepare into a Sanchi Tongshu Capsule of enteric pellet type, which can either ensure the function of enteric drug release of panaxatriol saponins, or overcome the quality problems that the enteric capsules are prone to dryness and brittle fracture, etc.

To realize the purpose of the present invention, one aspect of the present invention relates to an enteric pellet of panaxatriol saponins, primarily for use in the treatment of cardiovascular and cerebrovascular embolic diseases, comprising:
a) a drug core consisting of panaxatriol saponins and a pharmaceutically acceptable carrier; and
b) an enteric coating layer coating the drug core;
wherein,
the panaxatriol saponins, the pharmaceutically acceptable carrier and the enteric coating layer are in a weight ratio of 100:(165-208):(45-65);
the panaxatriol saponins per 100 mg comprise at least 50 mg of ginsenoside Rg1 (C₄₂H₇₂O₁₄), at least 6 mg of ginsenoside Re (C₄₈H₈₂O₁₈), at least 11 mg of notoginsenoside R1 (C₄₇H₈₁O₂₀); and
the drug core has a diameter of 20-24 mesh.

The pharmaceutically acceptable carrier of the panaxatriol saponins enteric pellet according to the present invention comprises an excipient and a binder.

The excipient may be any pharmaceutically called excipient, comprising microcrystalline cellulose, starch, dextrin, lactose, chitosan, mannitol, and micronized silica.

Preferably, the excipient consists of microcrystalline cellulose and starch, and the microcrystalline cellulose and starch are in a weight ratio of 1:0.5-1:1.5, more preferably 1:1.

Preferably, the excipient consists of microcrystalline cellulose and micronized silica, and the microcrystalline cellulose and micronized silica are in a weight ratio of 1:1.

The binder may be any pharmaceutically called binder, comprising hydroxymethyl cellulose, povidone (PVP), starch slurry, dextrin, and mucilage, wherein preferred binder comprises povidone K30 (PVPK30).

Preferably, the pharmaceutically acceptable carrier in the panaxatriol saponins enteric pellet according to the present invention consists of microcrystalline cellulose, starch, povidone K30, and the microcrystalline cellulose, starch and povidone K30 are in a weight ratio of (80-100):(80-100):(5-6).

Preferably, the pharmaceutically acceptable carrier in the panaxatriol saponins enteric pellet according to the present invention consists of microcrystalline cellulose, micronized silica, and povidone K30, and the microcrystalline cellulose, micronized silica and povidone K30 are in a weight ratio of 100:100:8.

Preferably, the enteric pellet consists of panaxatriol saponins : microcrystalline cellulose : starch : povidone K30 : enteric coating layer in a weight ratio of 100:93:93:5.7:58.3.

Preferably, the enteric pellet consists of panaxatriol saponins : microcrystalline cellulose : micronized silica: povidone K30 : enteric coating layer in a weight ratio of 100:100:100:8:62.

The panaxatriol saponins enteric pellet according to the present invention can employ any existing coating materials which have the function of enteric positioning drug release.

Preferably, the panaxatriol saponins according to the present invention are prepared by the following preparation method:
1. Crude medicine of Panax notoginseng is taken and crushed into coarsest powders, and is wetted evenly by 60-80% ethanol, placed hermetically for 6-10 hours, then filled into a percolator;
2. Percolation is performed by 3-7 times of 50-70% ethanol according to the volume of the coarsest powders of the crude medicine of Panax notoginseng filled in the percolator (hereinafter referred to as bed volume for short), and the flow rate of percolation is controlled at 0.15-0.30 time of bed volume per hour, and the percolate is collected;
3. It is concentrated under reduced pressure into an extractum with a relative density of 1.05-1.10 under a vacuum degree of 0.04-0.09 MPa, a temperature of 55-75°C, and ethanol is recovered;
4. The above extractum is diluted with water for 2-5 times, filtered with the temperature of the filtrate being controlled at 10-40°C, loaded onto a resin column with the rate being 0.25-0.50 time of the column volume per hour, and the column is kept for 2-8 hours after the loading;
5. It is eluted with water in 1.0-4.0 times of column volume, the flow rate is at first controlled at 0.25-0.75 time of column volume per hour for 30 minutes, then controlled at 0.50-1.00 time of column volume per hour for 30 minutes, and finally controlled at 1.00-1.50 times of column volume per hour until the completion of the elution;
6. It is eluted with 30-50% ethanol in 1.0-4.0 times of column volume, the flow rate is controlled at 0.25-0.75 time of column volume per hour, and the eluate is collected;
7. It is concentrated under reduced pressure into an extractum with a relative density of 1.08-1.12 under a vacuum degree of 0.04-0.09 MPa, a temperature of 55-75°C, and ethanol is recovered;
8. The above extractum is dried, to obtain panaxatriol saponins.

The panaxatriol saponins basically consist of ginsenosides Rg1, Re, and notoginsenoside R1, wherein the content of ginsenoside Rg1 is 30-60wt%, the content of ginsenoside Re is 4-15wt%, the content of notoginsenoside R1 is 5-20wt%, and the total content of the ginsenosides Rg1, Re, and notoginsenoside R1 is 39-95wt%.

More preferably, the panaxatriol saponins according to the present invention are prepared by the following preparation method:
1. Crude medicine of Panax notoginseng is taken and crushed into coarsest powders, and is wetted evenly by 65-75% ethanol, placed hermetically for 7-9 hours, then filled into a percolator;
2. Percolation is performed by 4-6 times of 55-65% ethanol according to the volume of the coarsest powders of the crude medicine of Panax notoginseng filled in the percolator (hereinafter referred to as bed volume for short), and the flow rate of percolation is controlled at 0.18-0.27 time of bed volume per hour, and the percolate is collected;
3. It is concentrated under reduced pressure into an extractum with a relative density of 1.05-1.10 under a vacuum degree of 0.04-0.09 MPa, a temperature of 55-75°C, and ethanol is recovered;
4. The above extractum is diluted with water for 2.5-4.5 times, filtered with the temperature of the filtrate being controlled at 10-35°C, loaded onto a resin column with the rate being 0.30-0.45 time of the column volume per hour, and the column is kept for 2-6 hours after the loading;
5. It is eluted with water in 1.5-3.5 times of column volume, the flow rate is at first controlled at 0.35-0.65 time of column volume per hour for 30 minutes, then controlled at 0.60-0.90 time of column volume per hour for 30 minutes, and finally controlled at 1.10-1.40 times of column volume per hour until the completion of the elution;
6. It is eluted with 35-45% ethanol in 1.5-3.5 times of column volume, the flow rate is controlled at 0.35-0.60 time of column volume per hour, and the eluate is collected;
7. It is concentrated under reduced pressure into an extractum with a relative density of 1.08-1.12 under a vacuum degree of 0.04-0.09 MPa, a temperature of 55-75°C, and ethanol is recovered;
8. The above extractum is dried, to obtain panaxatriol saponins.

The panaxatriol saponins basically consist of ginsenosides Rg1, Re, and notoginsenoside R1, wherein the content of ginsenoside Rg1 is 40-55wt%, the content of ginsenoside Re is 5-10wt%, the content of notoginsenoside R1 is 8-15wt%, and the total content of the ginsenosides Rg1, Re, and notoginsenoside R1 is 53-80wt%.

Most preferably, the panaxatriol saponins according to the present invention are prepared by the following preparation method:
1. Crude medicine of Panax notoginseng is taken and crushed into coarsest powders, and is wetted evenly by 70% ethanol, placed hermetically for 8 hours, then filled into a percolator;
2. Percolation is performed by 5 times of 60% ethanol according to the volume of the coarsest powders of the crude medicine of Panax notoginseng filled in the percolator (hereinafter referred to as bed volume for short), and the flow rate of percolation is controlled at 0.22 time of bed volume per hour, and the percolate is collected;
3. It is concentrated under reduced pressure into an extractum with a relative density of 1.05-1.10 under a vacuum degree of 0.04-0.09 MPa, a temperature of 55-75°C, and ethanol is recovered;
4. The above extractum is diluted with water for 4 times, filtered with the temperature of the filtrate being controlled at 30°C, loaded onto a resin column with the rate being 0.375 time of the column volume per hour, and the column is kept for 3 hours after the loading;
5. It is eluted with water in 2.5 times of column volume, the flow rate is at first controlled at 0.5 time of column volume per hour for 30 minutes, then controlled at 0.75 time of column volume per hour for 30 minutes, and finally controlled at 1.25 times of column volume per hour until the completion of the elution;
6. It is eluted with 40% ethanol in 2.5 times of column volume, the flow rate is controlled at 0.5 time of column volume per hour, and the eluate is collected;
7. It is concentrated under reduced pressure into an extractum with a relative density of 1.08-1.12 under a vacuum degree of 0.04-0.09 MPa, a temperature of 55-75°C, and ethanol is recovered;
8. The above extractum is dried, to obtain panaxatriol saponins.

The panaxatriol saponins basically consist of ginsenosides Rg1, Re, and notoginsenoside R1, wherein the content of ginsenoside Rg1 is 50-55wt%, the content of ginsenoside Re is 5-7wt%, the content of notoginsenoside R1 is 10-12wt%, and the total content of the ginsenosides Rg1, Re, and notoginsenoside R1 is 65-74wt%.

In the above preparation method of panaxatriol saponins, the resin is a styrene-type macroporous adsorption resin, and the model is selected from D101, D140, HPD100, etc., preferably D101; and the diameter to height ratio of the macroporous adsorption resin column is 1:3-6, preferably 1:4.

In another aspect, the present invention relates to a method for preparing the enteric pellet. The pellet preparation technologies studied and reported in recent years mostly employ centrifugal granulation method, extrusion-spheronization process, and fluidized bed process.

The fluidized bed process is placing drugs and carriers in a fluidized bed, blowing in airflow, mixing both of them evenly, then spraying the binder to form pellets, stopping spraying when the pellet size meet the requirements, and the obtained pellets can be dried directly in a boiling bed, and the coating procedures can be preformed at the same time, i.e., the granulation, the drying, the coating are completed in one step. During the whole procedures, the pellets have been always in a fluidized state, which can effectively prevent the occurrence of adhesion during the coating procedure. The advantages thereof are: the operating time is short; the product is homogeneous in size, round, narrow in particle size distribution, without adhesion; and the pellet coating layer is homogeneous in thickness. The extrusion-spheronization process is adding powders of drugs and carriers into the binder, and mixing them evenly to prepare soft materials, and extruding them into columnar shape by an extruder, and then cutting and rolling the cylindrical materials in a spheronizer into regular pellets with homogeneous size, and finally performing drying and coating. The obtained granules are homogeneous in size, narrow in particle size distribution, and homogeneous in drug content.

Therefore, the panaxatriol saponins enteric pellet according to the present invention is preferably prepared using the fluidized bed process or the extrusion-spheronization process.

The particular preparation steps of the fluidized bed process are: mixing the panaxatriol saponins as active principle and the excipient into homogeneous powders; formulating the binder into a solution in ethanol; spraying the mixed powders and the ethanol solution of the binder into a fluidized bed, to prepare initial cores having a diameter of 35-50 mesh; placing into the screened fluidized bed and further spraying the above mixed powders and ethanol solution of the binder to prepare the pellet, drying, removing, and screening the pellets of 20-24 mesh; placing them into a fluidized bed, and spraying the coating material for the enteric coating layer, to prepare the enteric pellets.

The particular preparation steps of the extrusion-spheronization process are: mixing the panaxatriol saponins as active principle and the excipient into homogeneous powders; formulating the binder into a solution in ethanol; adding the above ethanol solution of binder into the above mixed powders, to prepare soft materials; then extruding the soft materials by a extrusion-spheronizer into strips, then cutting the strips and spheronizing them into regular pellets with homogeneous size of 20-24 mesh, drying, and removing; placing the obtained pellets into the fluidized bed, coating with the enteric coating material, to prepare the enteric pellets.

In another aspect, the present invention also relates to a capsule comprising the enteric pellets. After drying and removing the above-prepared enteric pellets, filling them into empty capsules, to prepare into the Sanchi Tongshu Capsule of enteric pellet type according to the present invention. The empty capsule here may be any pharmaceutically called empty capsule, including empty gastric capsule and empty enteric capsule; because the panaxatriol saponins enteric pellets according to the present invention have possessed the function of enteric drug releasing and the gastric capsules have a relatively stable quality in the currently commercially available capsules, the preferred empty capsule of the capsule according to the present invention is the empty gastric capsule. The quality controlling method of the capsule is as follows:
1. Description: the present capsule is an enteric capsule, and the contents are pink pellets, and are light brown yellow after removing the coating; odorless, bitter in taste.
2. Identification: in the HPLC chromatogram recorded under the item "content determination", the test article should have peaks that are identical to the retention times of the reference substances of ginsenoside Rg1, ginsenoside Re, and notoginsenoside R1.
3. Inspection:
   Releasing rate: taking the present capsule, operating according to the releasing rate determination method (method 1 of the Second method in Appendix XD, "Chinese Pharmacopoeia" 2005 Edition, Part II), employing devices for dissolution determination method (the First method in Appendix XC, "Chinese Pharmacopoeia" 2005 Edition, Part II, 250 ml dissolution cup), 187.5 ml 0.1 mol/L hydrochloric acid solution is used as releasing media, and the rotating rate is 100 rpm, operating accordingly, after 120 minutes, both the enteric pellets and the releasing media are not allowed to have any obvious discoloration; then adding 62.5 ml 0.2 mol/L sodium phosphate solution which has been preheated to 37°C into the operating vessel, keeping the rotating rate constant, operating accordingly, sampling, preparing a solution of the test article, determining the contents of ginsenoside Rg1, ginsenoside Re and notoginsenoside R1 according to the method below the item of "content determination", calculating the dissolution amount of the 3 ingredients respectively, and the limits for the dissolution amounts of the 3 ingredients per capsule are respectively not allowed to be less than 70% of their content limitations.
   Others: It should accord with various related regulations under the item of capsule (Appendix I L, "Chinese Pharmacopoeia" 2005 Edition, Part I).
4. Content determination: determining according to HPLC method (Appendix VID, "Chinese Pharmacopoeia" 2005 Edition, Part I).

Chromatographic condition and systematic adaptability test: octadecyl silane bonded silica gel is used as filler; acetonitrile-water (19.5:80.5) is used as mobile phase; and the detection wavelength is 210 nm. The number of theoretical plates should not be less than 2000 calculated according to the peak of ginsenoside Rg1. The resolution between ginsenoside Rg1 and notoginsenoside R1 is not less than 1.3, and the resolution between ginsenosides Rg1 and Re is not less than 1.3.

Preparation of reference substance solution: taking appropriate amount of reference substances of ginsenoside Rg1, ginsenoside Re and notoginsenoside R1, which have been dried over phosphorus pentoxide desiccant for 24 hours, weighing precisely, adding mobile phase to dissolve and dilute them into a mixed solution comprising 0.4 mg ginsenoside Rg1, 0.06 mg ginsenoside Re and 0.06 mg notoginsenoside R1 per 1 ml, to obtain.

Preparation of test article solution: taking the content under the item of content uniformity, porphyrizing, mixing evenly, taking about 100 mg, weighing precisely, placing into a 500 ml measuring flask, adding appropriate amount of mobile phase, ultrasonic processing (power 150 W, frequency 40 kHz) for 15 minutes, and diluting to the scale mark, shaking well, filtering, and discarding the initial filtrate, taking the subsequent filtrate as the test article solution.

Determination method: precisely sucking 10 µl reference substance solution and test article solution respectively, and injecting into liquid chromatograph respectively, determining, to obtain.

The present capsule should comprise per capsule at least 50 mg of ginsenoside Rg1 (C₄₂H₇₂O₁₄), at least 6 mg of ginsenoside Re (C₄₈H₈₂O₁₈), and at least 11 mg of notoginsenoside R1 (C₄₇H₈₁O₂₀).

Pellets as a modem dosage form have obvious advantages in the aspect of pharmaceutical preparation studies, the pellets have nice appearance, good flowability, homogeneous size, small weight variation, and are easy to be filled into capsules; and are firm inside, and have a large drug content; and are very easy to be coated, and can reduce the contact with outside air, thus can effectively improve the hygroscopicity of the Chinese materia medica, and increase the stability of the drugs; and the pellets have a vast distribution area in the gastrointestinal tract, and are easy to be absorbed.

In the present invention, the enteric film coatings are coated after the panaxatriol saponins have been prepared into pellets, rather than employing particles to coat the enteric film coatings, which can avoid the non-homogeneous enteric film coating during coating due to the major differences in the particle size and shape, resulting in a poor controlling effects for the drug dissolution, thus affecting the effects of taking the drug by patients; while for employing the pellet coating, the shape is more homogeneous, and the coating agent can be coated more homogeneously onto the drug surface when being coated, by methods of controlling the size and roundness of pellet, which is conducive to the enteric effects, and to greatly increase the stability.

The panaxatriol saponins enteric pellets according to the present invention have had a function of enteric drug releasing, and can be filled directly into a common gastric capsule, i.e. can isolate the contact of drugs with light ray, air, resulting in antioxidant, moistureproof, and lucifugal effects, avoiding the degradation of the ingredients of panaxatriol saponins, to improve the stability of drugs, and are easy for storage and transportion.

The panaxatriol saponins enteric pellets according to the present invention are prepared into a Sanchi Tongshu Capsule of enteric pellet type, which can either control the good and stable release of the active ingredients of panaxatriol saponins within the small intestine, or can thoroughly solve the problems of the fragile enteric capsule and unstable quality, and for those who suffer from dysphagia, they can directly open the capsule and swallow the contents, thus ensure that the capsules according to the present invention to achieve good therapeutic effects.

The enteric pellets capsules have efficacies of promoting blood circulation for removing blood stasis, regulating meridians and collaterals, improving cerebral infarction and cerebral ischemic dysfunction, restoring ischemic cerebral metabolic abnormalities, anti-platelet aggregation, preventing thrombosis, improving microcirculation, reducing whole blood viscosity, increasing blood flow of carotid artery, and are primarily used in cardiovascular and cerebrovascular embolic diseases, indications: stroke, hemiplegia, askew mouth and tongue, sluggish speech, hemianesthesia.

### DESCRIPTION OF THE DRAWINGS

Figure 1. Fingerprints of panaxatriol saponins and the capsules of enteric pellet type according to the present invention.
Figure 2. Fingerprints of the capsules of enteric pellet type according to the present invention and commercially available Sanchi Tongshu Capsules.
Figure 3. Fingerprints of the capsules of enteric pellet type according to the present invention at month 0 and month 18.
Figure 4. Moisture adsorption curves of the capsules of enteric pellet type according to the present invention and commercially available Sanchi Tongshu Capsules.
Figure 5. Blood concentration-time curves for the in vivo absorption of the capsules of enteric pellet type according to the present invention in Beagles.

### DETAILED EMBODIMENTS

The present invention is further illustrated through the description of detailed embodiments below.

The percentage described in the present invention refers to weight percentage (g/g), unless otherwise specified.

### Example 1. Preparation example 1 of the panaxatriol saponins according to the present invention

Universal pulverizer (purchased from Shanghai Tianhe Pharmaceutical Machinery Factory, Model GF-300) was used to crush 150 kg dry rhizome of Panax notoginseng (place of origin: Wenshan Prefecture, Yunnan Province) into coarsest powders with a particle size range of 10-24 mesh, the above Panax notoginseng coarse powders were wet mixed with 65wt% ethanol, placed hermetically for 8 hours, and filled into a percolator; percolation was performed by 5 times of 65wt% ethanol according to the volume of the coarsest powders of the crude medicine of Panax notoginseng filled in the percolator (hereinafter referred to as bed volume for short), and the flow rate of percolation was controlled at 0.22 time of bed volume per hour, and the percolate was collected; the ethanol in the percolate was recovered under reduced pressure to obtain an alcoholic extractum of total Panax notoginseng saponins. The above alcoholic extractum of total Panax notoginseng saponins was formulated with purified water into a solution of 4 times of the crude medicine weight, filtered with the temperature of the filtrate being controlled at 30°C, loaded onto a styrene-type macroporous resin D140 (purchased from Chengdu Chenguang Chemical Research Institute) column with the rate being 0.375 time of the column volume per hour, and the column was kept for 3 hours after the loading; it was eluted with water in 2.5 times of column volume, the flow rate was at first controlled at 0.5 time of column volume per hour for 30 minutes, then controlled at 0.75 time of column volume per hour for 30 minutes, and finally controlled at 1.25 times of column volume per hour until the completion of the elution; it was eluted with 40% ethanol in 2.5 times of column volume, the flow rate was controlled at 0.5 time of column volume per hour, and the eluate was collected; it was concentrated under reduced pressure into an extractum with a relative density of 1.09 under a vacuum degree of 0.06 MPa, a temperature of 60°C, and ethanol was recovered; the above extractum was spray-dried, to obtain 9 kg panaxatriol saponins.

The Rg1, Re and R1 contents in the PTS obtained in Example 1 were determined to be 55wt%, 7wt% and 12wt%, respectively, by HPLC method, employing octadecyl silane bonded silica gel as filler, acetonitrile-water (volume ratio was 19.5:80.5) as mobile phase, and a detection wavelength of 210 nm.

### Example 2. Preparation example 2 of the panaxatriol saponins according to the present invention

Universal pulverizer (purchased from Shanghai Tianhe Pharmaceutical Machinery Factory, Model GF-300) was used to crush 100 kg dry rhizome of Panax notoginseng (place of origin: Wenshan Prefecture, Yunnan Province) into coarsest powders with a particle size range of 10-24 mesh, the above Panax notoginseng coarse powders were wet mixed with 70wt% ethanol, placed hermetically for 5 hours, and filled into a percolator; percolation was performed by 6 times of 60wt% ethanol according to the volume of the coarsest powders of the crude medicine of Panax notoginseng filled in the percolator (hereinafter referred to as bed volume for short), and the flow rate of percolation was controlled at 0.2 time of bed volume per hour, and the percolate was collected; the ethanol in the percolate was recovered under reduced pressure to obtain an alcoholic extractum of total Panax notoginseng saponins. The above alcoholic extractum of total Panax notoginseng saponins was formulated with purified water into a solution of 3 times of the crude medicine of weight, filtered with the temperature of the filtrate being controlled at 25°C, loaded onto a styrene-type macroporous resin D101 (purchased from Tianjin Pesticide Factory) column with the rate being 0.45 time of the column volume per hour, and the column was kept for 5 hours after the loading; it was eluted with water in 3 times of column volume, the flow rate was at first controlled at 0.6 time of column volume per hour for 30 minutes, then controlled at 0.9 time of column volume per hour for 30 minutes, and finally controlled at 1.4 times of column volume per hour until the completion of the elution; it was eluted with 45% ethanol in 3 times of column volume, the flow rate was controlled at 0.3 time of column volume per hour, and the eluate was collected; it was concentrated under reduced pressure into an extractum with a relative density of 1.10 under a vacuum degree of 0.08 MPa, a temperature of 70°C, and ethanol was recovered; the above extractum was spray-dried, to obtain 6 kg panaxatriol saponins.

The Rg1, Re and R1 contents in the PTS obtained in Example 1 were determined to be 50wt%, 6wt% and 11wt%, respectively, by HPLC method, employing octadecyl silane bonded silica gel as filler, acetonitrile-water (wolume ratio was 19.5:80.5) as mobile phase, and a detection wavelength of 210 nm.

### Example 3. Drug core formulation of the enteric pellets according to the present invention

Various components in the drug core formulation of the enteric pellets according to the present invention, their dosages and the determination results of evaluation indicators are shown in Table 1.

The evalutation indicators in the technology studies of the formulation according to the present invention:
1. The content determination of panaxatriol saponins:
   Refer to the contents of "content determination" in the quality control method of the enteric pellets according to the present invention.
2. Total transference rate of major avtive ingredients of panaxatriol saponins:
   Total transference rate=[(ginsenoside Rg1 amount + ginsenoside Re amount + notoginsenoside R1 amount after the technology)/(ginsenoside Rg1 amount + ginsenoside Re amount + notoginsenoside R1 amount before the technology)] × 100%
3. Physical indicators of pellets in the investigation of pellets compactibility:
   Physical indicators include roundness, friability, bulk density, angle of repose.
      (1) Roundness: 5 g pellets were placed on a plate, and one side of the plate was lifted to measure the included angle between the inclined plane and the horizontal plane when the pellets start to roll, the smaller the angel was, the roundness of the pellets was better.
      (2) Friability: 10 g pellets were weighed and placed into the small box of a tablet friability tester, and additional 5 silica gel spheres (small spheres with knock-on effects equipped in the tablet friability tester) were put in, and the pellets were taken out after oscillating for 5 min, screened with 24 mesh sieve, the weight (w) of pellets left on the sieve were weighed, to calculate the friability [Fr=(10-w)/w].
      (3) Bulk density: 5 g pellets were weighed, and placed into a 10 mL measuring cylinder, and were dropped from 5 cm away from the desktop for 3 consecutive times, the volume (V) thereof was determined, and the bulk density (d) was worked out from the formula d=m/V.
      (4) Angle of repose: The magnitude of angle of repose could reflect the flowability of the pellets, meanwhile could also indirectly reflect their roundness. The present test employed the fixed funnel method to determine.

### Yield of pellets:

Yield of pellets was calculated by the ratio of the weight of qualified pellets to the feeding amount.

**Table 1. Various components in the drug core formulation of the enteric pellets according to the present invention, their dosages and the determination results of evaluation indicators**

| Formulation No. | 1 | 2 | 3 | 4 | 5 |
|---|---|---|---|---|---|
| Powders of panaxatriol saponins (g) | 100 | 100 | 100 | 100 | 100 |
| Microcrystalline cellulose (g) | 100 | 80 | 93 | 80 | 100 |
| Starch (g) | 100 | 85 | 93 | 80 | - |
| Micronized silica gel (g) | - | - | - | - | 100 |
| Concentration of aqueous ethanol solution (%, ml/ml) | 50 | 50 | 50 | 50 | 50 |
| Volume of aquous ethanol solution (ml) | 70 | 70 | 70 | 70 | 100 |
| PVPK30 (g) | 5.7 | 5 | 5.7 | 5 | 8 |
| Drug loading capacity (%) | 33 | 38 | 35 | 38 | 33 |
| Pellet-forming nature | Good | Relatively good | Good | Relatively good | Good |
| Roundness (a) | 13.7 | 13.9 | 13.8 | 14.0 | 14.4 |
| Angle of repose (a) | 26.2 | 25.4 | 25.3 | 25.8 | 25.6 |
| Bulk density (g.mL⁻¹) | 0.685 | 0.687 | 0.660 | 0.688 | 0.734 |
| Friability (%) | 0.12 | 0.09 | 0.08 | 0.10 | 0.15 |
| R1 content (%) | 4.08 | 4.77 | 4.37 | 4.85 | 3.71 |
| Rg1 content (%) | 16.47 | 18.51 | 16.96 | 19.03 | 17.16 |
| Re content (%) | 2.80 | 2.91 | 2.83 | 3.10 | 1.98 |
| Total transference rate (%) | 92.94 | 87.20 | 91.26 | 86.32 | 88.70 |
| Yield of Pellets (%) | 95.02 | 90.05 | 94.57 | 88.12 | 92.00 |

The determination results of the test technology indicators of the above formulations suggested that the above 5 formulations could all be prepared into drug cores with relatively good pellet-forming nature, having relatively good physical indicators of pellets and relatively high yield of pellets. For the drug loading capacity of panaxatriol saponins, due to the relatively large viscosity of the materials of the panaxatriol saponins as active principle, relatively good pellet-forming nature could only be obtained when the drug loading capacity is ≤38%, and at the moment the yields of pellets were all relatively high as well; however, in consideration that larger dosage taken by a patient could be easily caused if the drug loading capacity was too small and the production costs could be higher, thus the drug loading capacity of the panaxatriol saponins enteric pellets according to the present invention was preferably 25%-38%.

### Example 4. Weight ratio of the drug core to the enteric coating layer of the enteric pellets according to the present invention (coating weight gain)

The enteric pellets according to the present invention could employ the coating system of Shanghai Colorcon Coating Technology Co., Ltd. as coating materials. The optimal coating weight gain was determined by comparing the releasing rates of the enteric pellets according to the present invention and the contents of the three active ingredients of ginsenosides Rg1, Re and notoginsenoside R1 in the panaxatriol saponins, under conditions of different coating weight gains. Each test employed 100 g pellets, with three weight gain levels of 18%, 20%, and 22%, to observe the indicators of coating weight gain, and the test results are shown in Table 2.

**Table 2. Screening results of different coating weight gains**

| Serial number | Coating weigh gain (%) | R1 content (%) | Rg1 content (%) | Re content (%) | Total yield (%) | Total transference (%) | Releasing rate in acid | | | Releasing rate in buffer | | |
|---|---|---|---|---|---|---|---|---|---|---|---|---|
| | | | | | | | R1 | Rg1 | Re | R1 | Rg1 | Re |
| 1 | 18 | 3.48 | 14.48 | 2.36 | 99.83 | 99.12 | 0 | 0 | 0 | 86.09 | 84.85 | 83.08 |
| 2 | 20 | 3.45 | 14.29 | 2.28 | 99.75 | 99.16 | 0 | 0 | 0 | 84.86 | 83.30 | 73.16 |
| 3 | 22 | 3.39 | 14.44 | 2.21 | 99.43 | 100.64 | 0 | 0 | 0 | 66.88 | 63.66 | 59.12 |

It can be seen from the above table that, when the coating weight gain is 18%-22%, the contents of ginsenosides Rg1, Re and notoginsenoside R1 and the releasing rate values can achieve the quality requirements.

### Example 5. Preparation of the enteric pellets according to the present invention and the capsules comprising the same using fluidized bed process

The present Example was based on the formulation 3 of Example 3, and the enteric coating layer materials of Example 4 were employed, and the panaxatriol saponins in the formulation were prepared by following the methods of Example 1 or Example 2 according to the present invention, and other carriers and packaging materials were all commercially available:
Microcrystalline cellulose: purchased form Huzhou Zhanwang Pharmaceutical Co., Ltd. (Batch No.: 20070454);
Starch: purchased from Jining Six Best Excipients Co., Ltd. (Batch No.: 20070601);
Povidone: purchased from Huzhou Zhanwang Pharmaceutical Co., Ltd. (Batch No.: 20070201);
Enteric coating premix: purchased from Shanghai Colorcon Coating Technology Co., Ltd. (Batch No.: THL005545);
Empty capsule: purchased from Suzhou Capsugel Ltd. (Batch No.: 12830301).

Preparation equipment: Chongqing Guangxia CPM-300 testing type fluidized pellet preparation coating machine (technological parameters for the equipment were: temperature of the materials 30°C, atomization pressure 0.15 MPa, air seal pressure 0.08 MPa, liquid supplying rate 2.8 HZ, drying time 30 minutes).

### Specific preparation technology:

100 g panaxatriol saponins in the formulation 3 were taken, and mixed with 93 g starch, 93 g microcrystalline cellulose into homogeneous powders, for later use. Additional 5.7 g PVPK30 was taken, and added into 70 ml 50% (ml/ml) aqueous ethanol solution, to formulate into a binder. The above mixed powders and the above binder were sprayed into a fluidized bed respectively, to prepare 35-50 mesh initial cores, which were placed in the fluidized bed after screening, and the spraying of the mixed powders and the binder was continued to prepare the pellets, which were dried, and taken out, screened for 20-24 mesh pellets, and placed in the fluidized bed, and about 58.3 g enteric film coating materials in the Example 4 were sprayed in, to prepare the enteric pellets, which were dried, taken out, and filled into the capsules, to prepare 1000 capsules (0.35 g/capsule, each capsule comprising 100 mg panaxatriol saponins).

### Example 6. Preparation of the enteric pellets according to the present invention and the capsule comprising the same using extrusion-spheronization process

The present Example was based on the formulation 5 of Example 3, and the enteric coating layer materials of Example 4 were employed, and the panaxatriol saponins in the formulation were prepared by following the methods of Example 1 or Example 2 according to the present invention, and other carriers and packaging materials were all commercially available:
Microcrystalline cellulose: purchased form Huzhou Zhanwang Pharmaceutical Co., Ltd. (Batch No.: 20070454);
Micronized silica: purchased form Huzhou Zhanwang Pharmaceutical Co., Ltd. (Batch No.: 20081201);
Povidone: purchased from Huzhou Zhanwang Pharmaceutical Co., Ltd. (Batch No.: 20070201);
Enteric coating premix: purchased from Shanghai Colorcon Coating Technology Co., Ltd. (Batch No.: THL005545);
Empty capsule: purchased from Suzhou Capsugel Ltd. (Batch No.: 12830301).

### Preparation equipment:

1. Chongqing Enger E-50 testing type extrusion-spheronizer set (technological parameters for the equipment were: extruding speed 30 rpm, spheronizing speed 900 rpm, spheronizing time 1 minute, drying time 30 minutes);
2. Chongqing Guangxia CPM-300 testing type fluidized pellet preparation coating machine (technological parameters for the equipment were: temperature of the materials 30°C, atomization pressure 0.15 MPa, air seal pressure 0.08 MPa, liquid supplying rate 2.8 HZ, drying time 30 minutes).

### Specific preparation technology:

100 g panaxatriol saponins, microcrystalline cellulose, micronized silica in the formulation 5 were taken respectively and mixed into homogeneous powders, for later use. Additional 8g PVPK30 was taken, added into 100 ml 50% (ml/ml) aqueous ethanol solution, to formulate into a binder. The above binder was added into the above powders, to prepare soft materials, then the soft materials were extruded by the extrusion-spheronizer, first into columnar shape, then the columnar shape materials were cut, rolled into 20-24 mesh regular pellets with homogeneous size, which were dried and taken out. The prepared pellets were placed into a fluidized bed, and about 62 g enteric film coating materials of Example 4 were sprayed in, to prepare the enteric pellets, which were dried, taken out, filled into capsules, to prepare 1000 capsules (0.37 g/capsule, each capsule comprising 100 mg panaxatriol saponins).

### Example 7. Formulation comparison between the capsule of enteric pellet type according to the present invention and the commercially available Sanchi Tongshu Capsule

The formulation of the capsule of enteric pellet type according to the present invention was the formulation of Example 5.

The formulation of the commercially available Sanchi Tongshu Capsule was: panaxatriol saponins 100 g, starch 100 g, to prepare 1000 capsules (0.2 g/capsule, each capsule comprising 100 mg panaxatriol saponins).

The contents of pharmaceutically effective ingredients per unit preparation contained in both the formulations remained unchanged, both were that each capsule contained 100 mg panaxatriol saponins, thus the usage and dosage and curative effects of the drugs were not affected.

### Example 8. Preparation technology comparison between the capsule of enteric pellet type according to the present invention and the commercially available Sanchi Tongshu Capsule

The technology of the capsule of enteric pellet type according to the present invention was the technology of Example 5.

The technology of the commercially available Sanchi Tongshu Capsule was: 100 g panaxatriol saponins were taken, dissolved in 20 ml 50% (ml/ml) aqueous ethanol solution, and 100 g starch was added, mixed, and wet granulated employing fluidized bed granulator, dried under reduced pressure, crushed, filled into the capsules, to prepare 1000 capsules.

In comparison with the commercially available Sanchi Tongshu Capsule, the preparation method of the capsule of enteric pellet type according to the present invention primarily increased the steps for the pellets preparation and the pellets coating technology, but these steps were all completed in the fluidized bed coating pellet preparation machine, all of them were physical processes of spraying coating, the temperature of materials was controlled below 40°C, and the conditions were mild, thus would not adversely affect the drugs.

### Example 9. Comparative studies of fingerprints between the capsule of enteric pellet type according to the present invention and the commercially available Sanchi Tongshu Capsule

Three batches of capsules of enteric pellet type according to the present invention were prepared according to the formulation and technology of Example 5, having batch numbers of A01, A02, A03, respectively, and three batches of commercially available Sanchi Tongshu Capsules were obtained from Pharmaceutical Factory of Chengdu Huasun Group Inc., LTD, having batch numbers of 080304, 080305, 080306.

The fingerprints of samples of the three batches of capsules of enteric pellet type according to the present invention, the three batches of commercially available Sanchi Tongshu Capsules, the raw materials panaxatriol saponins, as well as the fingerprints of the three batches of capsules of enteric pellet type according to the present invention after long-term placement for 18 months (the temperature was 25°C±2°C, the humidity was 60±10%) were determined, to investigate the effects of the preparation technological process on the pharmaceutical material basis, to investigate whether the pharmaceutical material bases of the enteric pellet type according to the present invention and the commercially available Sanchi Tongshu Capsules were identical, and to investigate whether the pharmaceutical material basis of the enteric pellet type according to the present invention after long-term placement was stable.

### 1. The determination conditions for the fingerprints

Refer to the determination conditions for the fingerprint of panaxatriol saponins (panaxatriol saponins was recorded in the drug standard of the "Chinese Pharmacopoeia" 2010 Edition), to perform the fingerprint determination.

### 2. The results of the fingerprint determination and analyses

### (1) The fingerprint comparison between panaxatriol saponins and the capsule of enteric pellet type according to the present invention:

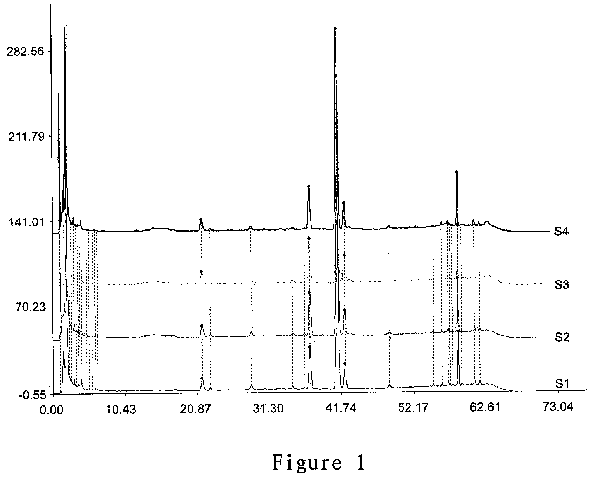

Each fingerprint was compared according to the similarity evaluation system of Chinese materia medica chromatography fingerprint, and the results are shown in Figure 1. Wherein, S1 is the fingerprint of panaxatriol saponins, and S2-S4 are the fingerprints of the 3 batches of the capsule of enteric pellet type according to the present invention, A01, A02, A03.

The evaluation results of similarities between the raw material panaxatriol saponins and the 3 batches of the capsule of enteric pellet type according to the present invention were: 0.988 for batch No. A01, 0.963 for batch No. A02, 0.957 for batch No. A03. Thus, it could be seen that the similarities of the comparison between the fingerprints of the three batches of the capsules of enteric pellet type according to the present invention respectively and the fingerprint of their raw material panaxatriol saponins were all above 0.9, indicating that the pharmaceutical material basis of the capsule of enteric pellet type according to the present invention was not changed during the technology production.

### (2) The comparison between the fingerprints of the capsule of enteric pellet type according to the present invention and the commercially available Sanchi Tongshu Capsule

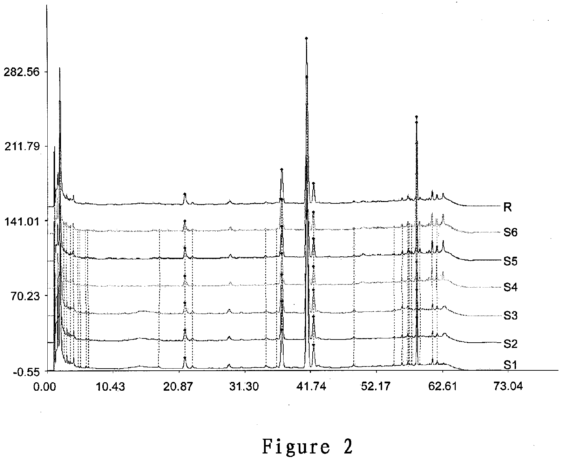

Each fingerprint was compared according to the similarity evaluation system of Chinese materia medica chromatography fingerprint, and the results are shown in Figure 2. Wherein, R is the control fingerprint, generated by S1-S6; S1-S3 are the fingerprints of the 3 batches of the capsule of enteric pellet type according to the present invention, A01, A02, A03, respectively; S4-S6 are the fingerprints of the 3 batches of commercially available Sanchi Tongshu Capsule, 080304, 080305, 080306, respectively. The evaluation results of similarities between the capsule of enteric pellet type according to the present invention and the commercially available Sanchi Tongshu Capsule are shown in Table 3:

**Table 3. The evaluation results of similarities between the capsule of enteric pellet type according to the present invention and the commercially available Sanchi Tongshu Capsule**

| Specification | The capsule of enteric pellet type according to the present invention | | | The commercially available Sanchi Tongshu Capsule | | |
|---|---|---|---|---|---|---|
| Batch No. | A01 | A02 | A03 | 080304 | 080305 | 080306 |
| The similarity between the control fingerprint and each batch | 0.975 | 0.976 | 0.970 | 0.975 | 0.949 | 0.949 |

It can be seen from Table 3 that the similarities of the comparison between the capsules of enteric pellet type according to the present invention and the commercially available Sanchi Tongshu Capsule are all above 0.9, indicating that the pharmaceutical material basis of the capsules of enteric pellet type according to the present invention is identical to that of the commercially available Sanchi Tongshu Capsule.

### (3) The comparison between fingerprints of the capsules of enteric pellet type according to the present invention at month 0 and 18.

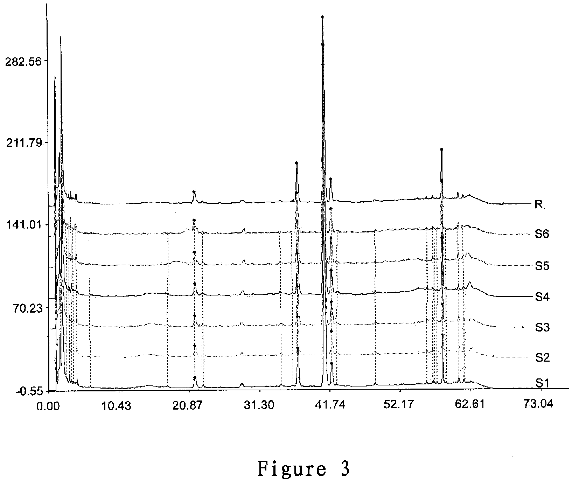

Each fingerprint was compared according to the similarity evaluation system of Chinese materia medica chromatography fingerprint, and the results are shown in Figure 3. Wherein, R is the control fingerprint, generated by S1-S6; S1-S3 are the fingerprints of the 3 batches of the capsule of enteric pellet type according to the present invention at month 0, A01, A02, A03, respectively; S4-S6 are the fingerprints of the 3 batches of the capsule of enteric pellet type according to the present invention at month 18, A01, A02, A03, respectively. The evaluation results of similarities of the capsule of enteric pellet type according to the present invention at month 0 and 18, are shown in Table 4:

**Table 4. The evaluation results of similarities of the capsule of enteric pellet type according to the present invention at month 0 and 18**

| Month | Month 0 | | | Month 18 | | |
|---|---|---|---|---|---|---|
| Batch No. | A01 | A02 | A03 | A01 | A02 | A03 |
| The similarity between the control fingerprint and each batch | 0.990 | 0.988 | 0.983 | 0.988 | 0.968 | 0.990 |

It can be seen from the above table that the similarities of the comparison between the capsules of enteric pellet type according to the present invention at month 0 and month 18 are all above 0.9. This indicates that the pharmaceutical material basis of the capsules of enteric pellet type according to the present invention after long-term placement is not changed.

### Example 10. The comparison between the hygroscopicity of capsules of enteric pellet type according to the present invention and the commercially available Sanchi Tongshu Capsule

### 1. Comparison of moisture absorption rate

The contents of the capsule of enteric pellet type according to the present invention prepared according to the formulation and technology of Example 5 and the commercially available Sanchi Tongshu Capsule, were subjected to hygroscopicity studies; the test articles were precisely weighed into weighing bottles, then placed for 7 days to reach a balanced state under conditions of a relative humidity of 75%±2%, and a temperature of 25°C±2°C, then the test articles were weighed again, to obtain the equilibrium moisture absorption content under this relative humidity; the data are shown in Table 5.

**Table 5. Data of moisture absorption rate study**

| Sample | Weight of the weighing bottle (g) | Weight of the weighing bottle + sample (g) | Weight of the smple (g) | Weight of the weighing bottle + sample after moisture absorption (g) | Moisture (g) | Moisture absorption rate (%) | Average moisture absorption rate (%) |
|---|---|---|---|---|---|---|---|
| The enteric pellet 1 according to the present invention | 13.7750 | 15.7870 | 2.0120 | 15.9014 | 0.1144 | 5.69 | 5.59 |
| The enteric pellet 2 according to the present invention | 12.4705 | 14.7542 | 2.2837 | 14.8795 | 0.1253 | 5.49 | |
| Content 1 of the commercially available Capsule | 19.469 | 21.4141 | 1.9451 | 21.5674 | 0.1533 | 7.88 | 7.38 |
| Content 2 of the commercially available Capsule | 21.0174 | 23.1486 | 2.1312 | 23.3164 | 0.1678 | 6.88 | |

Compared to the contents of the commercially available Sanchi Tongshu Capsule, the hygroscopicity of the capsule of enteric pellet type according to the present invention significantly decreased.

### 2. Moisture absorption curve

The capsule of enteric pellet type according to the present invention prepared according to the formulation and technology of Example 5 and the contents of the commercially available Sanchi Tongshu Capsule were respectively placed in airtight desiccators with different relative humidity of 30%-100%, to obtain the equilibrium moisture absorption rates under various relative humidity; the results are shown in Table 6.

**Table 6. Data of the moisture absorption curve study**

| Solution type | MgCl₂ | K₂CO₃ | NaBr | Kl | NaCl | KCl | KNO₃ |
|---|---|---|---|---|---|---|---|
| Relative humidity (%) | 32.78 | 43.16 | 59.57 | 68.86 | 75.29 | 80.89 | 92.50 |
| The moisture absorption rate of the capsule of enteric pellet type according to the present invention (%) | 1.84 | 1.92 | 2.83 | 4.21 | 5.59 | 7.11 | 10.64 |
| The moisture absorption rate of the contents of the commercially available capsules (%) | 1.12 | 1.82 | 2.44 | 5.33 | 14.55 | 23.8 | 33.28 |

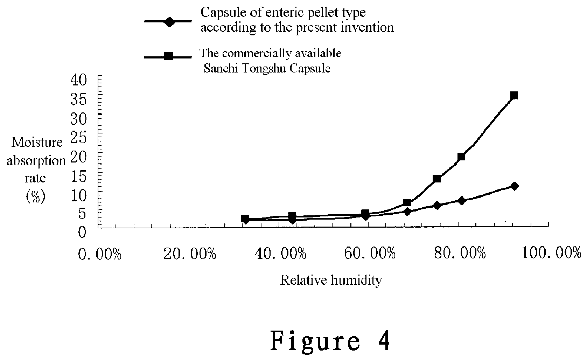

Figure was plotted using moisture absorption rate as vertical coordinate and relative humidity as horizontal coordinate, to obtain a moisture absorption curve, which is shown in Figure 4.

The above tests suggested that the hygroscopicity of the drug was decreased after the contents of the capsules were changed into enteric pellets, which could secure the drug stability better.

### Example 11. The comparative study of the in vitro releasing rates between the capsule of enteric pellet type according to the present invention and the commercially available Sanchi Tongshu Capsule

According to the requirements of releasing amount of enteric preparation in the "Chinese Pharmacopoeia" 2005 Edition, Part II that "the releasing amount in acid should not excess 10% of the labelled content, and the releasing amount in the sustained-releasing solution should not below 70% of the labelled content", the rotating basket method of method 1 in the Second method of releasing rate determination method according to the "Chinese Pharmacopoeia" 2005 Edition, Part II was used to perform the examination of releasing rate of ginsenosides Rg1, Re and notoginsenoside R1.

The samples of the three batches of the capsule of enteric pellet type according to the present invention (batch numbers were A01, A02, A03, respectively) of Example 5 and the three batches of the commercially available Sanchi Tongshu Capsule (batch numbers were 080304, 080305, 080306, respectively) were taken to perform the comparative studies of in vitro releasing rates, and the results are shown in Table 7.

**Table 7. Results of the releasing rate determination of the capsule of enteric pellet type according to the present invention and the commercially available Sanchi Tongshu Capsule**

| Batch No. | Releasing rate of ginsenoside Rg1 (%, x̅ ± SD, n=6) | | Releasing rate of ginsenoside Re (%, x̅ ± SD, n=6) | | Releasing rate of notoginsenoside R1 (%, x̅ ± SD, n=6) | |
|---|---|---|---|---|---|---|
| | In acid | In buffering solution | In acid | In buffering solution | In acid | In buffering solution |
| A01 | 0 | 89.73±0.70 | 0 | 92.26±0.24 | 0 | 87.61±1.49 |
| A02 | 0 | 90.35±0.32 | 0 | 93.71±0.69 | 0 | 88.11±0.78 |
| A03 | 0 | 88.68±0.64 | 0 | 90.84±1.03 | 0 | 89.47±0.46 |
| 080304 | 0 | 94.90±0.76 | 0 | 95.84±0.95 | 0 | 93.13±6.94 |
| 080305 | 0 | 94.37±0.53 | 0 | 94.73±0.52 | 0 | 88.05±3.97 |
| 080306 | 0 | 94.40±1.11 | 0 | 94.66±0.43 | 0 | 84.14±1.30 |

The results suggested: the releasing rates of the capsule of enteric pellet type according to the present invention and the commercially available Sanchi Tongshu Capsule in acid were all <10%, and in the sustained-releasing solution were all >70%, which all met the requirements of the enteric preparation, without significant difference.

### Example 12. The comparative studies of in vivo absorption process of the capsule of the enteric pellet type according to the present invention and the commercially available Sanchi Tongshu Capsule in Beagles

### 1. Objective of the test

Beagles were used as test subjects, while the ginsenoside Rg1, ginsenoside Re and notoginsenoside R1 were used as testing indicators; the in vivo absorption processes of the capsule of enteric pellet type according to the present invention and the commercially available Sanchi Tongshu Capsule in animals were compared, to establish a "blood concentration-time" curve, and comparative studies on the main pharmaceutical ingredients, the bioequivalence in the in vivo absorption process in animals of both the specifications of Sanchi Tongshu Capsules were performed, to evaluate the rationality of the formulation and technology.

### 2. Instruments, test drugs, and animals

### 2.1. Instruments

Agilent 1100 HPLC (Agilent 1100 UV detector; Quaternary pump online degassing system; Agilent 1100 chromatographic working station) (Agilent technologies co., LTD, US);
Waters Symetry Shield C18 column (4.6 mm×250 mm, 5 µm) (Waters Corporation);
Mettler AE240 hundred thousandth electronic balance (Mettler Corporation, Germany);
KQ3200 ultrasonic cleaner (Kun Shan Ultrasonic Instruments Co ., Ltd);
GM-0.33II diaphragm vacuum pump (Tianjin Jinteng Co.,Ltd);
Thermostat water bath (General Factory of Shanghai Medical Instruments);
WH-3 micro vortex mixing apparatus (Shanghai Hu Xi Analysis Instrument Factory);
TDL-5 desk centrifuge (Shanghai Anting Scientific Instrument Factory).

### 2.2. Reagents and test drugs

The capsule of enteric pellet type according to the present invention of Example 5 (Batch No. A01);
Commercially available Sanchi Tongshu Capsule (Pharmaceutical Factory of Chengdu Huasun Group Inc., LTD, Batch No.: 080902);
Notoginsenoside R1 standard (Batch No.: 110745-200415), ginsenoside Rg1 standard (Batch No.: 110703-200726), ginsenoside Re (Batch No.: 110754-200421), astragaloside reference substance (hereinafter referred to as IS, Batch No.: 110781-2006113), all of which were purchased from the National Institute For the Control of Biological of China;
UR050SH heparin sodium vacuum blood collection tube (Zhejiang U-REAL Medical Technology Co.,Ltd, Batch No.: 0810001);
Vacuum blood collector supporting needle (Shanghai Kangnong Medical Devices Co., Ltd., Batch No.: 20080909);
0.9% sodium chloride injection (Sichuan Kelun Pharmaceutical Co., Ltd.);
Acetonitrile (chromatographic pure, Fisher Corporation);
Ultrapure water (self-made in the laboratory).

### 2.3. Laboratory animals

6 healthy Beagles, male, body weight 10 kg±2 kg, were provided by Istitute of Laboratory Aminal, Sichuan Academy of Medical Sciences. They were raised in dog's house of ordinary house in the Laboratory Animal Center of Chengdu University of Traditional Chinese Medicine, in single cage respectively, the rearing conditions met the requirements of national standard of GB 14925-2001, the ambient temperature was 19°C±3°C, the relative humidity was 55%±15%, and the dog feed was purchased from the Jianyang Base of Laboratory Aminal, Sichuan Academy of Medical Sciences. The quality certificate number of the laboratory animal was SCXK(Chuan)2006-024.

### 3. Method for determining the HPLC contents of R1, Rg1, Re in Beagle plasma samples

The main pharmaceutically effective ingredients of the capsule of enteric pellet type according to the present invention and the commercially available Sanchi Tongshu Capsule were all R1, Rg1 and Re, and the contents of which were relatively high, and they were also the main material basis for the pharmaceutical efficacy of both the specifications, so that R1, Rg1 and Re were used as detection subjects and evaluation indicators in the tests of in vivo absorption of drugs in animals.

### 3.1. Preparation of the reference substance solution

Stock solution of R1 reference substance: appropriate amount of R1 reference substance was taken, precisely weighed, and placed into a 10 ml measuring flask, dissolved with distilled water and diluted to the scale mark, shook well, to obtain, and the concentration was about 1.6 mg/ml.

Stock solution of Rg1 reference substance: appropriate amount of Rg1 reference substance was taken, precisely weighed, and placed into a 10 ml measuring flask, dissolved with distilled water and diluted to the scale mark, shook well, to obtain, and the concentration was about 1.5 mg/ml.

Stock solution of Re reference substance: appropriate amount of Re reference substance was taken, precisely weighed, and placed into a 10 ml measuring flask, dissolved with distilled water and diluted to the scale mark, shook well, to obtain, and the concentration was about 0.6 mg/ml.

Stock solution of mixed reference substances: stock solution of R1 reference substance, stock solution of Rg1 reference substance, and stock solution of Re reference substance were taken for 1 ml each, and placed into 5 ml measuring flask, diluted with distilled water and diluted to the scale mark, shake well, to obtain.

Stock solution of astragaloside (IS) reference substance: appropriate amount of astragaloside reference substance was taken, precisely weighed, and placed into a 10 ml measuring flask, dissolved with methanol and diluted to the scale mark, to prepare a 0.60 mg/ml stock solution of internal standard.

The above stock solutions of reference substances were all placed in a refrigerator for cold storage (the stable period for the stock solution was 1 month), and diluted before use.

### 3.2. Chromatographic conditions

Percentages (%) referred in the chromatographic conditions were all volume percentages (ml/ml).

Chromatographic column Waters Symetry Shield C18 column (4.6 mm×250 mm, 5µm); C18 pre-column; mobile phase was acetonitrile-water gradient elution: 0→25 min, acetonitrile 21%→23%, water 79%→77%; 25→30 min, acetonitrile 23%→40%, water 77%→60%; 30→45 min, acetonitrile 40%→45%, water 60%→55%; flow rate: 1.0 ml/min; column temperature: 35°C; detection wavelength 203 nm; sample size 20 µl, running time 45 min.

### 3.3. Pretreatment method for plasma samples

Methanol acetonitrile precipitation method was employed, the ratio between plasma and methanol, acetonitrile was 2:1:2, and the internal standard was astragaloside.

Preparation method for dog plasma samples: 1.0 ml plasma sample was precisely measured, and added sequentially with 0.1 ml solution of astragaloside reference substance, 0.5 ml methanol, 1.0 ml acetonitrile, and vortexed for 3 min, centrifuged at 15000 rpm for 3 min, and the supernatant was separated, evaporated in a 40°C bath by nitrogen gas, and the residue was dissolved in 350 µl methanol, and vortexed for 30 s, centrifuged at 15000 rpm for 3 min, and the supernatant was taken, to obtain.

### 4. Comparative studies of in vivo absorption in animal

### 4.1. Test method

### (1) Medication method for animals

6 Beagles were randomly divided into two groups of A and B (3 for each group), two-period crossover design was employed, to perform the test by self-control method, and the wash-out period was 7 days. They were fasted for 12 hours before the medication, and 5 ml blank blood sample was drawn from femoral vein 15 min before the medication; uniform meals (commercially available dog feed) were taken 5 h after the medication, and water was taken uniformly.

First cycle: Group A were orally given 10 capsules of the commercially available Sanchi Tongshu Capsule, and Group B were orally given 10 capsules of the capsule of enteric pellet type according to the present invention, and blood was collected at different time points. Group A: 3 ml blood sample was collected from the femoral vein of the lower limbs of dogs at 0.5 h, 1 h, 1.5 h, 2 h, 2.5 h, 3 h, 3.5 h, 4 h, 6 h, 10 h, 12 h, 16 h, 20 h, 24 h, 14 sampling points altogether; Group B: 3 ml blood sample was collected from the femoral vein of the lower limbs of dogs at 0.5 h, 1 h, 1.5 h, 2 h, 2.5 h, 3 h, 3.5 h, 4 h, 6 h, 10 h, 12 h, 16 h, 20 h, 24 h, 14 sampling points altogether; the blood samples were uniformly stored in heparinized plastic centrifuge tubes, and plasma was separated by centrifuging at 150000 rpm for 3 min, and the plasma was stored at an environment of -20°C to be tested.

Second cycle: Group A were given 10 capsules of the capsule of enteric pellet type according to the present invention and Group B were given 10 capsules of the commercially available Sanchi Tongshu Capsule, and the blood collection points were the same to the first cycle.

### (2) Content determination for Rg1, Rg1, Re in the plasma

Beagle plasma samples were taken, and the solutions of plasma test articles were prepared according to the preparation method of the test article, and 20 µl of each plasma test article was precisely measured, injected into liquid chromatograph, to record chromatograms. Internal standard method was employed, and the contents of R1, Rg1, Re in the plasma were calculated by an accompanying standard curve. Blood concentrations were calculated, and the blood concentrations from 6 Beagles were averaged, and the average blood concentrations of both drugs were compared, and the blood concentration-time curves were plotted.

### 4.2. Test results

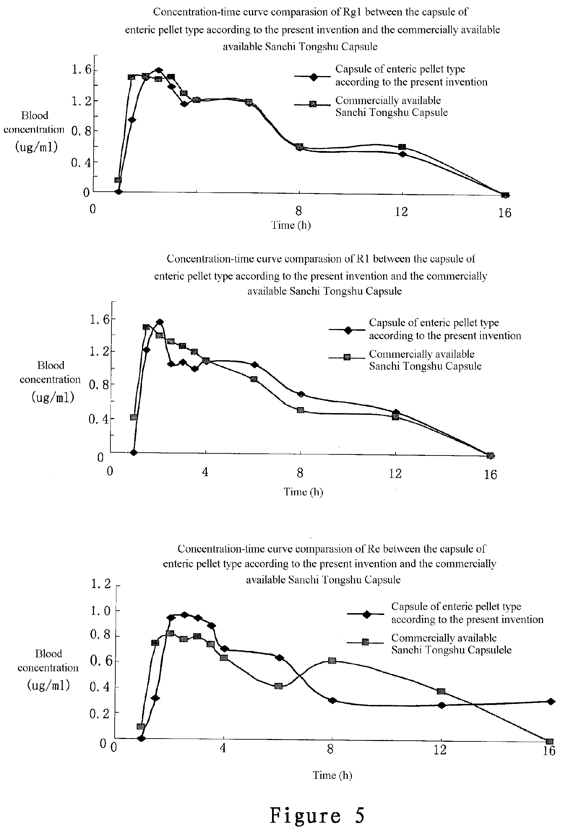

The results of the average in vivo blood concentrations of R1, Rg1, and Re of the capsule of enteric pellet type according to the present invention and the commercially available Sanchi Tongshu Capsule in 6 dogs measured in the tests are shown in Table 8, and the blood concentration-time curves are shown in Figure 5.

**Table 8. Average in vivo blood concentrations of R1, Rg1, and Re of the capsule of enteric pellet type according to the present invention and the commercially available Sanchi Tongshu Capsule in 6 dogs (µg/ml)**

| Time(h) | The capsule of enteric pellet type according to the present invention | | | The Commercially Available Sanchi Tongshu Capsule | | |
|---|---|---|---|---|---|---|
| | R1 | Rg1 | Re | R1 | Rg1 | Re |
| 1.0 | - | - | - | 0.4075 | 0.1479 | 0.0942 |
| 1.5 | 1.2258 | 0.9507 | 0.3154 | 1.4990 | 1.5008 | 0.7468 |
| 2 | 1.5638 | 1.5008 | 0.9482 | 1.3873 | 1.5302 | 0.8258 |
| 2.5 | 1.0576 | 1.6126 | 0.9737 | 1.3233 | 1.4852 | 0.7794 |
| 3 | 1.0753 | 1.3932 | 0.9512 | 1.2675 | 1.5171 | 0.7979 |
| 3.5 | 1.0010 | 1.1624 | 0.8900 | 1.1950 | 1.3044 | 0.7397 |
| 4 | 1.0891 | 1.2242 | 0.7066 | 1.1027 | 1.2106 | 0.6342 |
| 6 | 1.0566 | 1.1736 | 0.6445 | 0.8719 | 1.1971 | 0.4118 |
| 8 | 0.7072 | 0.6041 | 0.3076 | 0.5112 | 0.6252 | 0.6171 |
| 12 | 0.4946 | 0.5340 | 0.2825 | 0.4360 | 0.6265 | 0.3822 |
| 16 | 0.0000 | 0.0000 | 0.3154 | 0.0000 | 0.0000 | 0.0000 |

### 4.3. Data processing

### (1) Compartment model processing

The blood concentrations measured were input into a computer, and processed by 3P87 pharmacokinetic software statistical program, and distinguishments of the compartment model were performed by employing AIC method, and the parameters of the degree of fitting were shown in Table 9.

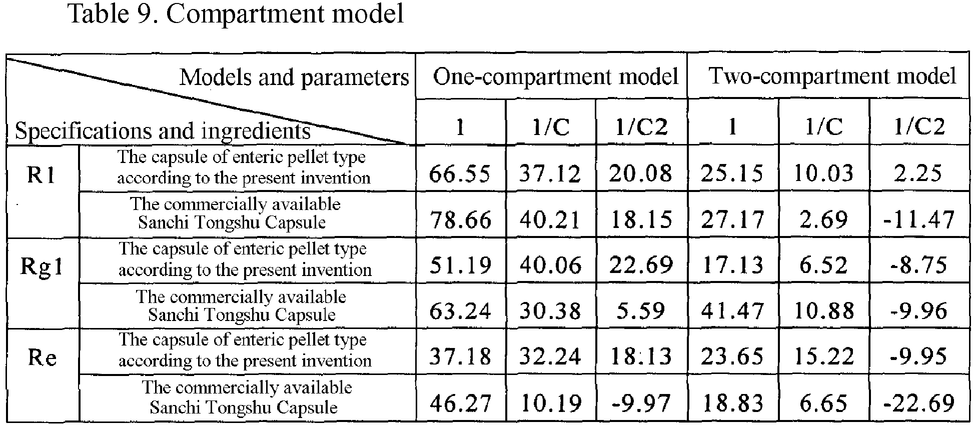

It can be seen from Table 9 that the in vivo absorption processes of the 3 main ingredients of the two specifications of capsules all have relatively good degree of fitting for two-compartment model, indicating that the in vivo absorption processes of the 3 main ingredients of the two specifications of the commercially available Sanchi Tongshu Capsule all conform to the characteristics of the two-compartment model.

### (2) The calculation of the pharmacokinetic parameters

After the oral medication of the two specifications of the commercially available Sanchi Tongshu Capsules, the calculated results of the pharmacokinetic parameters of the 3 main pharmaceutically effective ingredients are shown in Table 10. MRT was obtained by statistical moment method, and AUC0-t was obtained by trapezoidal method.

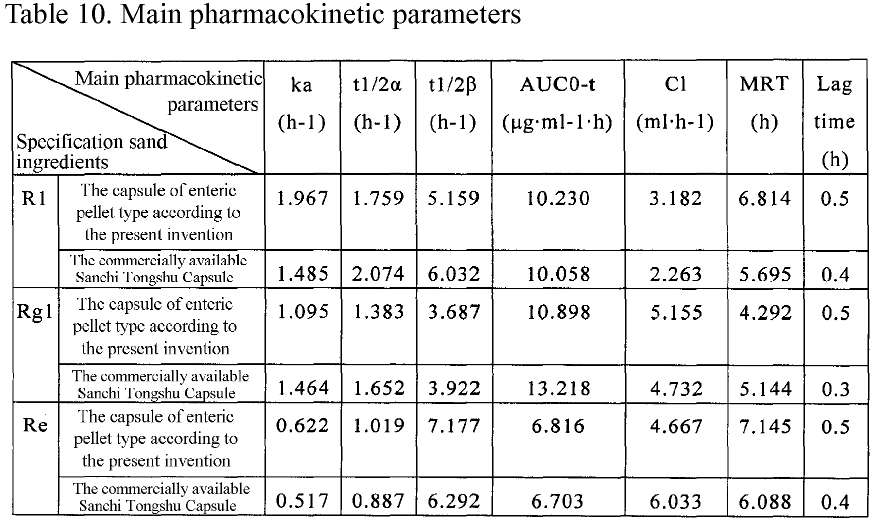

### (3) Result analysis

The in vivo absorption processes of the 3 main ingredients of the two specifications of the Sanchi Tongshu Capsule all conformed to the pharmacokinetic characteristics of the two-compartment model, one distribution phase (α-phase) and one elimination phase (β-phase) could be observed. t tests were performed to the pharmacokinetic parameters corresponding to the index ingredients in the two specifications of drugs respectively, and the results all had no significant differences (P>0.05), indicating that the 3 main pharmaceutically effective ingredients in the two specifications of Sanchi Tongshu Capsules have similar in vivo absorption processes, and close bioavailabilities.

### 5. Comparison of in vivo absorption processes of the commercially available Sanchi Tongshu Capsule and the capsule of enteric pellet type according to the present invention

The in vivo absorption processes of the 3 main pharmaceutical effective ingredients of the two specifications of Sanchi Tongshu Capsule all conformed to the pharmacokinetic characteristics of the two-compartment model, one distribution phase (α-phase) and one elimination phase (β-phase) could be observed. The two specifications of drugs are all enteric preparations, which do not release drugs in the stomach, and rapidly release drugs after entering into the small intestine, the pharmacokinetic parameters of the index ingredients thereof are all very close, there are no significant differences in the in vivo absorption processes, indicating that the in vivo absorption of the main ingredients in the two specifications of drugs are the same. t tests were performed for the AUC0-t values of R1 Rg1, and Re, corresponding to the index ingredients in the preparation of the two specifications respectively, and the results all have no significant differences (P>0.05), indicating that the bioavailabilities of both the specifications of drugs were equivalent.

It can be seen from the above tests that the capsule of enteric pellet type according to the present invention and the commercially available Sanchi Tongshu Capsule are all enteric preparations, which do not release drugs in the stomach, and rapidly release drugs after entering into the small intestine, the pharmacokinetic parameters of the index ingredients thereof are all very close, without significant differences, indicating that the in vivo processes of the main pharmaceutically effective ingredients in both of them are essentially equivalent.

### Example 13. The comparative study of the stabilities between the capsule of enteric pellet type according to the present invention and the commercially available Sanchi Tongshu Capsule

Stress tests, accelerated stability tests, and long term stability tests for the capsule of enteric pellet type according to the present invention and the commercially available Sanchi Tongshu Capsule were performed under equivalent conditions.

Three batches of the capsules of enteric pellet type according to the present invention (Batch numbers were A01, A02, A03, respectively) and three batches the commercially available Sanchi Tongshu Capsule (Batch numbers were 080304, 080305, 080306, respectively) were taken and stress tests, accelerated stability tests of 6 months, and long term stability tests of 18 months were performed under equivalent conditions, and the stabilities of the capsule of enteric pellet type according to the present invention were comparatively investigated.

Commercially available package, pharmaceutical PVC hard sheet/PTP aluminum foil for drug package, and carton package were used to pack the three batches of the capsules of enteric pellet type according to the present invention.

### Stress test

### 1.1 High temperature test

Samples of the above six batches of the capsules were taken, and placed in 60°C incubator under commercially available packages, and sampled to test at day 0 and day 10, the average values of each item of results are shown in Table 11.

### 1.2 Illumination test

Samples of the above six batches of the capsules were taken, and placed in 4500±500 Lx illumination box under commercially available packages, and sampled to test at day 0 and day 10, the average values of each item of results are shown in Table 11.

### 1.3 High humidity test

Samples of the above six batches of the capsules were taken, and placed in 92.5% relative humidity (KNO₃ saturated solution) desiccator under commercially available package, and were observed in a 25°C constant temperature incubator and sampled to test at day 0 and day 10, the average values of each item of results are shown in Table 11.

### 2. Accelerated stability test

Samples of the above six batches of the capsule were placed in conditions of a temperature of 40°C±2°C, a relative humidity of 75%±5%, for 6 months, and were sampled to test in month 0, month 1, month 2, month 3, month 6, respectively, wherein the results of test results at the end of the month 0, month 1, month 3, month 6 are shown in Table 12.

### 3. Long term stability test

Samples of the above six batches of the capsule were placed in conditions of a temperature of 25°C±2°C, a relative humidity of 60%±10%, and were sampled to test in month 0, month 3, month 6, month 9, month 12, month 18, respectively, wherein the test results at the end of the month 0, month 6, month 12, month 18 are shown in Table 13.

The above results suggested that the capsule of enteric pellet type according to the present invention and the commercially available Sanchi Tongshu Capsule all had no obvious changes under high temperature conditions, strong light conditions, high humidity conditions; after 6 months of accelerated test and 18 months of long term stability test, the results of their description, content uniformity, moisture, disintegration time, microbial limit, and contents determination, etc. also all had no obvious changes (test results of each time and average value of the results all conformed the standard requirements). However, at month 6 of the accelerated stability tests or after month 12 of the long term stability tests, some of the capsule shells of the commercially available Sanchi Tongshu Capsule were easily broken after slight pinch, and phenomena of increased moisture in the capsule contents occurred, meanwhile the capsule of enteric pellet type according to the present invention had no such phenomena.

The results of the stability studies suggested that, compared to the commercially available Sanchi Tongshu Capsule, the capsule of enteric pellet type according to the present invention could improve the hygroscopicity of the contents, and solve the frangibility problem of the original capsule shell, so that the stability was better.

**Table 11. The stress test results of the capsule of enteric pellet type according to the present invention and the commercially available Sanchi Tongshu Capsule**

| Investigated items | Standard requirement | The capsule of enteric pellet type according to the present invention | | | | The commercially available Sanchi Tongshu Capsule | | | |
|---|---|---|---|---|---|---|---|---|---|
| | | Day 0 | High temperature Day 10 | Strong linght Day 10 | High humidity Day 10 | Day 0 | High temperature Day 10 | Strong linght Day 10 | High humidity Day 10 |
| Apperance | Enteric capsule, the contents were pink pellets, light brown after removing the coating odorless, bitter in taste. | Conform the regulations | Conform the regulations | Conform the regulations | Conform the regulations | Conform the regulations | Conform the regulations | Conform the regulations | Conform the regulations |
| Content uniformity | Should comform the regulations | Conform the regulations | Conform the regulations | Conform the regulations | Conform the regulations | Conform the regulations | Conform the regulations | Conform the regulations | Conform the regulations |
| Disintegration time or releasing rate | Should comform the regulations | Conform the regulations | Conform the regulations | Conform the regulations | Conform the regulations | Conform the regulations | Conform the regulations | Conform the regulations | Conform the regulations |
| Moisture | ≤9.0% | 5.47% | 5.37% | 5.40% | 5.53% | 6.57% | 6.67% | 6.70% | 6.87% |
| identification | Chromatographic peaks of corresponding to the reference substances ofginsenoside Rg1, ginsenoside Re and notoginsenoside R1 should be detected | Detected | Detected | Detected | Detected | Detected | Detected | Detected | Detected |
| Content | Ginsenoside Rg1 (C42H72O14) should not be less than 50 mg per capsule | 52.60 | 52.73 | 52.73 | 52.73 | 54.40 | 54.40 | 54.60 | 53.67 |
| | Ginsenoside Re (C48H82O18) should not be less than 6 mg per capsule | 8.93 | 9.03 | 9.07 | 9.03 | 7.07 | 7.03 | 7.10 | 7.03 |
| | Notoginsenoside R1 (C47H81O20) should not be less than 11 mg per capsule | 1323 | 11.30 | 11.30 | 11.30 | 1333 | 13.37 | 13.53 | 13.50 |
| Microbial limit | Bacterial counts (≤1000/g) | <10/g | <10/g | <10/g | <10/g | <10/g | <10/g | <10/g | <10/g |
| | Mold counts (<100/g) | <10/g | <10/g | <10/g | <10/g | <10/g | <10/g | <10/g | <10/g |
| | E. coli (shall not be detected/g) | Not detected/g | Not detected/g | Not detected/g | Not detected/g | Not detected/g | Not detected/g | Not detected/g | Not detected/g |

**Table 12. The accelerated stability test results of the capsule of enteric pellet type according to the present invention and the commercially available Sanchi Tongshu Capsule**

| Investigated items | Standard requirements | The capsule of enteric pellet type according to the present invention | | | | The commercially available Sanchi Tongshu Capsule | | | |
|---|---|---|---|---|---|---|---|---|---|
| | | Accelerated Month 0 | Accelerated Month 1 | Accelerated Month 3 | Accelerated Month 6 | Accelerated Month 0 | Accelerated Month 1 | Accelerated Month 3 | Accelerated Month 6 |
| Apperance | Enteric capsule, the cotents were pink pellets, light brown after removing the coating; odorless, bitter in taste. | Conform the regulations | Conform the regulations | Conform the regulations | Conform the regulations | Conform the regulations | Conform the regulations | Conform the regulations | Conform the regulations |
| Content uniformity | Should comform the regulations | Conform the regulations | Conform the regulations | Conform the regulations | Conform the regulations | Conform the regulations | Conform the regulations | Conform the regulations | Conform the regulations |
| Disintegration time or releasing rate | Should comform the regulations | Conform the regulations | Conform the regulations | conform regulations | Conform the regulations | Conform the regulations | Conform the regulations | Conform the regulations | Conform the regulations |
| Moisture | ≤9.0% | **5.50%** | **5.70%** | **5.60%** | **5.47%** | **6.57%** | **6.80%** | **6.40%** | **7.73%** |
| Identification | Chromatographic peaks of corresponding to the reference substances of ginsenoside Rg1, ginsenoside Re and notoginsenoside R1, should be detected | Detected | Detected | Detected | Detected | Detected | Detected | Detected | Detected |
| Content | Ginsenoside Rg1 (C42H72O14) should not be less than 50 mg per capsule | 52.73 | 52.57 | 52.60 | 52.57 | 54.40 | 54.63 | 55.70 | 56.80 |
| | Ginsenoside Re (C48H82O18) should not be less than 6 mg per capsule | 9.03 | 8.60 | 9.30 | 9.27 | 7.07 | 7.07 | 7.00 | 7.03 |
| | Notoginsenoside R1 (C47H81O20) should not be less than 11 mg per capsule | 11.30 | 11.17 | 11.13 | 11.30 | 13.30 | 13.20 | 13.77 | 14.53 |
| Microbial limit | Bacterial counts (≤1000/g) | <10/g | <10/g | <10/g | <10/g | <10/g | <10/g | <10/g | <10/g |
| | Mold counts (≤100/g) | <10/g | <10/g | <10/g | <10/g | <10/g | <10/g | <10/g | <10/g |
| | E. coli (shall not be detected /g) | Not detected /g | Not detected /g | Not detected /g | Not detected /g | Not detected /g | Not detected /g | Not detected /g | Not detected /g |

**Table 13. The long term stability test results of the capsule of enteric pellet type according to the present invention and the commercially available Sanchi Tongshu Capsule**

| Investigated items | Standard requirements | The capsule of enteric pellet type according to the present invention | | | | The commercially available Sanchi Tongshu Capsule | | | |
|---|---|---|---|---|---|---|---|---|---|
| | | Long term Month 0 | Long term Month 6 | Long term Month 12 | Long term Month 18 | Long term Month 0 | Long term Month 6 | Long term Month 12 | Long term Month 18 |
| Apperance | Enteric capsule, the cotents were pink pellets, light brown after removing the coating; odorless, bitter in taste. | Conform the regulations | Conform the regulations | Conform the regulations | Conform the regulations | Conform the regulations | Conform the regulations | Conform the regulations | Conform the regulations |
| Content uniformity | Should comform the regulations | Conform the regulations | Conform the regulations | Conform the regulations | Conform the regulations | Conform the regulations | Conform the regulations | Conform the regulations | Conform the regulations |
| Disintegration time or releasing rate | Should comform the regulations | Conform the regulations | Conform the regulations | Conform the regulations | Conform the regulations | Conform the regulations | Conform the regulations | Conform the regulations | Conform the regulations |
| Moisture | **≤9.0%** | **5.50%** | **5.50%** | **5.47%** | **5.53%** | **6.57%** | **6.57%** | **6.83%** | **6.97%** |
| Identification | Chromatographic peaks of corresponding to the reference substances ofginsenoside Rg1, ginsenoside Re and notoginsenoside R1, should be detected | Detected | Detected | Detected | Detected | Detected | Detected | Detected | Detected |
| Content | Ginsenoside Rg1 (C42H72O14) should not be less than 50 mg per capsule | 52.73 | 53.03 | 52.60 | 51.87 | 54.40 | 55.67 | 55.30 | 53.73 |
| | Ginsenoside Re (C48H82O18) should not be less than 6 mg per capsule | 9.03 | 9.27 | 8.93 | 8.70 | 7.07 | 7.00 | 7.00 | 7.07 |
| | Notoginsenoside R1 (C47H81O20) should not be less than 11 mg per capsule | 11.30 | 11.33 | 13.23 | 12.63 | 13.30 | 14.03 | 13.67 | 13.47 |
| Microbial limit | Bacterial counts (≤1000/g) | <10/g | <10/g | <10/g | <10/g | <10/g | <10/g | <10/g | <10/g |
| | Mold counts (≤100/g) | <10/g | <10/g | <10/g | <10/g | <10/g | <10/g | <10/g | <10/g |
| | E. coli (shall not be detected/g) | Not detected/g | Not detected/g | Not detected/g | Not detected/g | Not detected/g | Not detected/g | Not detected/g | Not detected/g |

## Claims

1. An enteric pellet of panaxatriol saponins, comprising:
a) a drug core consisting of panaxatriol saponins and a pharmaceutically acceptable carrier; and
b) an enteric coating layer coating the drug core;
wherein,
the panaxatriol saponins, the pharmaceutically acceptable carrier and the enteric coating layer are in a weight ratio of 100:(165-208):(45-65);
the panaxatriol saponins per 100 mg comprise at least 50 mg of ginsenoside Rg1 (C₄₂H₇₂O₁₄), at least 6 mg of ginsenoside Re (C₄₈H₈₂O₁₈), at least 11 mg of notoginsenoside R1 (C₄₇H₈₁O₂₀); and
the drug core has a diameter of 20-24 mesh.

2. The enteric pellet according to claim 1, wherein the pharmaceutically acceptable carrier comprises an excipient and a binder.

3. The enteric pellet according to claim 2, wherein the excipient comprises one or more of microcrystalline cellulose, starch, dextrin, lactose, chitosan, mannitol, and micronized silica.

4. The enteric pellet according to claim 3, wherein the excipient consists of microcrystalline cellulose and starch, and the microcrystalline cellulose and the starch are in a weight ratio of 1:0.5-1:1.5.

5. The enteric pellet according to claim 4, wherein the microcrystalline cellulose and the starch are in a weight ratio of 1:1.

6. The enteric pellet according to claim 3, wherein the excipient consists of microcrystalline cellulose and micronized silica, and the microcrystalline cellulose and the micronized silica are in a weight ratio of 1:1.

7. The enteric pellet according to claim 2, wherein the binder comprises one or more of hydroxymethyl cellulose, povidone (PVP), starch slurry, dextrin, and mucilage.

8. The enteric pellet according to claim 7, wherein the binder is povidone K30 (PVPK30).

9. The enteric pellet according to claim 2, wherein the pharmaceutically acceptable carrier consists of microcrystalline cellulose, starch and povidone K30, and the microcrystalline cellulose, the starch and the povidone K30 are in a weight ratio of (80-100):(80-100):(5-6).

10. The enteric pellet according to claim 2, wherein the pharmaceutically carrier consists of microcrystalline cellulose, micronized silica and povidone K30, and the microcrystalline cellulose, the micronized silica and the povidone K30 are in a weight ratio of 100:100:8.

11. The enteric pellet according to claim 9, wherein the enteric pellet consists of panaxatriol saponins : microcrystalline cellulose : starch : povidone K30 : enteric coating layer in a weight ratio of 100:93:93:5.7:58.3.

12. The enteric pellet according to claim 10, wherein the enteric pellet consists of panaxatriol saponins : microcrystalline cellulose : micronized silica : povidone K30 : enteric coating layer in a weight ratio of 100:100:100:8:62.

13. A capsule of panaxatriol saponins, comprising the enteric pellet according to any of claims 1-12.

14. A method for preparing the enteric pellet according to any of claims 1-12, which is prepared by fluidized bed process, comprising the particular steps of:
1) providing homogeneous powders formed by mixing panaxatriol saponins as active principle and the excipient;
2) providing an solution of the binder in ethanol;
3) spraying the mixed powders and the ethanol solution of the binder into the fluidized bed, to prepare initial cores of 35-50 mesh;
4) placing the screened initial cores into the fluidized bed and further spraying the above mixed powders and the ethanol solution of the binder to prepare the pellets, drying, and removing;
5) screening the pellets of 20-24 mesh; placing them into a fluidized bed, and spraying the coating material for the enteric coating layer, to prepare the enteric pellets.

15. A method for preparing the enteric pellet according to any of claims 1-12, which is prepared by extrusion-spheronization process, comprising the particular steps of:
1) providing homogeneous powders formed by mixing panaxatriol saponins as active principle and the excipient;
2) providing a solution of the binder in ethanol;
3) adding the ethanol solution of the binder into the mixed powders, to prepare a soft material;
4) extruding the soft material through an extrusion-spheronizer into strips, then cutting the strips and spheronizing them into regular pellets with homogeneous size of 20-24 mesh, drying, and removing;
5) placing the obtained pellets into the fluidized bed, coating with the enteric coating material, to prepare the enteric pellets.

## Patentansprüche

1. Magensaftresistentes Pellet von Panaxatriol-Saponinen, umfassend:
a) einen Arzneimittelkern bestehend aus Panaxatriol-Saponinen und einem pharmazeutisch akzeptablen Träger; und
b) eine den Arzneimittelkern umhüllende magensaftresistente Beschichtung;
wobei,
die Panaxatriol-Saponine, der pharmazeutisch akzeptable Träger und die magensaftresistente Beschichtung in einem Gewichtsverhältnis von 100:(165-208):(45-65) vorliegen;
die Panaxatriol-Saponine pro 100 mg wenigstens 50 mg Ginsenosid Rg1 (C₄₂H₇₂O₁₄), wenigstens 6 mg Ginsenosid Re (C₄₈H₈₂O₁₈), wenigstens 11 mg Notoginsenosid R1 (C47H81O20) enthalten; und
der Arzneimittelkern einen Durchmesser von 20-24 Siebmaschenweite (Mesh) aufweist.

2. Magensaftresistentes Pellet nach Anspruch 1, wobei der pharmazeutisch akzeptable Träger einen Arzneistoffträger und ein Bindemittel umfasst.

3. Magensaftresistentes Pellet nach Anspruch 2, wobei der Arzneistoffträger eines oder mehrere von mikrokristalliner Cellulose, Stärke, Dextrin, Lactose, Chitosan, Mannit und mikronisierte Kieselsäure umfasst.

4. Magensaftresistentes Pellet nach Anspruch 3, wobei der Arzneistoffträger aus mikrokristalliner Cellulose und Stärke besteht und die mikrokristalline Cellulose und die Stärke in einem Gewichtsverhältnis von 1:0,5-1:1,5 vorliegen.

5. Magensaftresistentes Pellet nach Anspruch 4, wobei die mikrokristalline Cellulose und die Stärke in einem Gewichtsverhältnis von 1:1 vorliegen.

6. Magensaftresistentes Pellet nach Anspruch 3, wobei der Arzneistoffträger aus mikrokristalliner Cellulose und mikronisierter Kieselsäure besteht und die mikrokristalline Cellulose und die mikronisierte Kieselsäure in einem Gewichtsverhältnis von 1:1 vorliegen.

7. Magensaftresistentes Pellet nach Anspruch 2, wobei das Bindemittel eines oder mehrere von Hydroxymethylcellulose, Povidon (PVP), Stärkeaufschlämmung, Dextrin und Schleimstoff umfasst.

8. Magensaftresistentes Pellet nach Anspruch 7, wobei das Bindemittel Povidon K30 (PVPK30) ist.

9. Magensaftresistentes Pellet nach Anspruch 2, wobei der pharmazeutisch akzeptable Träger aus mikrokristalliner Cellulose, Stärke und Povidon K30 besteht, und die mikrokristalline Cellulose, die Stärke und das Povidon K30 in einem Gewichtsverhältnis von (80-100):(80-100):(5-6) vorliegen.

10. Magensaftresistentes Pellet nach Anspruch 2, wobei der pharmazeutische Träger aus mikrokristalliner Cellulose, mikronisierter Kieselsäure und Povidon K30 besteht, und die mikrokristalline Cellulose, die mikronisierte Kieselsäure und das Povidon K30 in einem Gewichtsverhältnis von 100:100:8 vorliegen.

11. Magensaftresistentes Pellet nach Anspruch 9, wobei das magensaftresistente Pellet aus Panaxatriol-Saponinen : mikrokristalliner Cellulose : Stärke : Povidon K30 : magensaftresistenter Beschichtung in einem Gewichtsverhältnis von 100:93:93:5,7:58,3 besteht.

12. Magensaftresistentes Pellet nach Anspruch 10, wobei das magensaftresistente Pellet aus Panaxatriol-Saponinen : mikrokristalliner Cellulose : mikronisierter Kieselsäure : Povidon K30 : magensaftresistenter Beschichtung in einem Gewichtsverhältnis von 100:100:100:8:62 besteht.

13. Kapsel mit Panaxatriol-Saponinen, umfassend das magensaftresistente Pellet nach einem der Ansprüche 1-12.

14. Verfahren zur Herstellung des magensaftresistenten Pellets nach einem der Ansprüche 1-12, das mittels Wirbelschichtverfahren hergestellt wird, umfassend die einzelnen Schritte von:
1) Bereitstellen homogener Pulver, die durch Mischen von Panaxatriol-Saponinen als aktives Prinzip und dem Arzneistoffträger gebildet wurden;
2) Bereitstellen einer Lösung des Bindemittels in Ethanol;
3) Sprühen des Pulvergemisches und der Ethanol-Lösung des Bindemittels in das Wirbelschichtbett, um Ausgangskerne von 35-50 Siebmaschenweite (Mesh) herzustellen;
4) Platzieren der ausgesiebten Ausgangskerne in das Wirbelschichtbett und weiteres Sprühen des obigen Pulvergemisches und der Ethanol-Lösung des Bindemittels, um die Pellets herzustellen, Trocknen und Entnehmen;
5) Identifizieren der Pellets von 20-24 Siebmaschenweite (Mesh); Platzieren derselben in ein Wirbelschichtbett, und Sprühen des Beschichtungsmaterials für die magensaftresistente Beschichtung, um die magensaftresistenten Pellets herzustellen.

15. Verfahren zur Herstellung des magensaftresistenten Pellets nach einem der Ansprüche 1-12, das mittels Extrusion-Sphäronisierung-Verfahren hergestellt wird, umfassend die einzelnen Schritte von:
1) Bereitstellen homogener Pulver, die durch Mischen von Panaxatriol-Saponinen als aktives Prinzip und dem Arzneistoffträger gebildet wurden;
2) Bereitstellen einer Lösung des Bindemittels in Ethanol;
3) Zugeben der Ethanol-Lösung des Bindemittels in die Pulvergemische, um ein weiches Material herzustellen;
4) Extrudieren des weichen Materials durch einen Extrusion-Sphäronisierer zu Streifen, dann Schneiden der Streifen und Sphäronisieren derselben zu regelmäßigen Pellets mit homogener Größe von 20-24 Siebmaschenweite (Mesh), Trocknen und Entnehmen;
5) Platzieren der erhaltenen Pellets in das Wirbelschichtbett, Beschichten mit dem magensaftresistenten Beschichtungsmaterial, um die magensaftresistenten Pellets herzustellen.

## Revendications

1. Granule gastro-résistant de saponines de panaxatriol, comprenant :
a) un noyau de médicament constitué de saponines de panaxatriol et d'un support pharmaceutiquement acceptable ; et
b) une couche d'enrobage gastro-résistant enrobant le noyau de médicament ;
dans lequel,
les saponines de panaxatriol, le support pharmaceutiquement acceptable et la couche d'enrobage gastro-résistant sont dans un rapport en poids de 100 : (165 à 208) : (45 à 65) ;
les saponines de panaxatriol pour 100 mg comprennent au moins 50 mg de ginsénoside Rg1 (C₄₂H₇₂P₁₄), au moins 6 mg de ginsénoside Re (C₄₈H₈₂O₁₈), au moins 11 mg de notoginsénoside R1 (C₄₇H₈₁O₂₀) ; et
le noyau de médicament a un diamètre de 20 à 24 mesh.

2. Granule gastro-résistant selon la revendication 1, dans lequel le support pharmaceutiquement acceptable comprend un excipient et un liant.

3. Granule gastro-résistant selon la revendication 2, dans lequel l'excipient comprend un ou plusieurs éléments parmi la cellulose microcristalline, l'amidon, la dextrine, le lactose, le chitosan, le mannitol, et la silice micronisée.

4. Granule gastro-résistant selon la revendication 3, dans lequel l'excipient est constitué de cellulose microcristalline et d'amidon, et la cellulose microcristalline et l'amidon sont dans un rapport en poids de 1 : 0,5 à 1 : 1,5.

5. Granule gastro-résistant selon la revendication 4, dans lequel la cellulose microcristalline et l'amidon sont dans un rapport en poids de 1 : 1.

6. Granule gastro-résistant selon la revendication 3, dans lequel l'excipient est constitué de cellulose microcristalline et de silice micronisée, et la cellulose microcristalline et la silice micronisée sont dans un rapport en poids de 1 : 1.

7. Granule gastro-résistant selon la revendication 2, dans lequel le liant comprend un ou plusieurs éléments parmi l'hydroxyméthylcellulose, la povidone (PVP), la boue d'amidon, la dextrine et le mucilage.

8. Granule gastro-résistant selon la revendication 7, dans lequel le liant est la povidone K30 (PVPK30).

9. Granule gastro-résistant selon la revendication 2, dans lequel le support pharmaceutiquement acceptable est constitué de cellulose microcristalline, d'amidon et de povidone K30, et la cellule microcristalline, l'amidon et la povidone K30 sont dans un rapport en poids de (80 à 100) : (80 à 100) : (5 à 6).

10. Granule gastro-résistant selon la revendication 2, dans lequel le support pharmaceutiquement acceptable est constitué de cellulose microcristalline, de silice micronisée et de povidone K30, et la cellulose microcristalline, la silice micronisée et la povidone K30 sont dans un rapport en poids de 100 : 100 : 8.

11. Granule gastro-résistant selon la revendication 9, dans lequel le granule gastro-résistant est constitué de saponines de panaxatriol : cellulose
microcristalline : amidon : povidone K30 : couche d'enrobage gastro-résistant dans un rapport en poids de 100 : 93 : 93 _{:} 5,7 : 58,3.

12. Granule gastro-résistant selon la revendication 10, dans lequel le granule gastro-résistant est constitué de saponines de panaxatriol : cellulose microcristalline : silice micronisée : povidone K30 : couche d'enrobage gastro-résistant dans un rapport en poids de 100 : 100 : 100 : 8 : 62.

13. Capsule de saponines de panaxatriol, comprenant le granule gastro-résistant selon l'une quelconque des revendications 1 à 12.

14. Procédé de préparation du granule gastro-résistant selon l'une quelconque des revendications 1 à 12, qui est préparé par un processus à lit fluidisé, comprenant les étapes particulières de :
1) fourniture de poudres homogènes formées par mélange de saponines de panaxatriol en tant que principe actif et de l'excipient ;
2) fourniture d'une solution du liant dans de l'éthanol ;
3) pulvérisation des poudres mélangées et de la solution d'éthanol du liant dans le lit fluidisé, pour préparer des noyaux initiaux de 35 à 50 mesh ;
4) placement des noyaux initiaux criblés dans le lit fluidisé et en outre pulvérisation des poudres mélangées ci-dessus et de la solution d'éthanol du liant pour préparer les granules, séchage et retrait ;
5) criblage des granules de 20 à 24 mesh ; leur placement dans un lit fluidisé, et pulvérisation de la matière d'enrobage pour la couche d'enrobage gastro-résistant, pour préparer les granules gastro-résistants.

15. Procédé de préparation du granule gastro-résistant selon l'une quelconque des revendications 1 à 12, qui est préparé par un processus d'extrusion-sphéronisation, comprenant les étapes particulières de :
1) fourniture de poudres homogènes formées par mélange de saponines de panaxatriol en tant que principe actif et l'excipient ;
2) fourniture d'une solution du liant dans de l'éthanol ;
3) addition de la solution d'éthanol du liant dans les poudres mélangées, pour préparer une matière molle ;
4) extrusion de la matière molle à travers une machine d'extrusion-sphéronisation en rubans, puis découpe des rubans et leur sphéronisation en granules réguliers de taille homogène de 20 à 24 mesh, séchage, et retrait ;
5) placement des granules obtenus dans le lit fluidisé, enrobage avec la matière d'enrobage gastro-résistant, pour préparer les granules gastro-résistants.
